# EUROPÄISCHE PATENTSCHRIFT

(11) **EP 0 704 438 B1**
(45) Veröffentlichungstag und Bekanntmachung des Hinweises auf die Patenterteilung: **11.02.1998**
(21) Anmeldenummer: 95112240.7
(22) Anmeldetag: 03.08.1995
(51) Int. Cl.: C07D 277/18

(54) **Verfahren zur Herstellung von 2-Cyaniminothiazolidin**
Process for the preparation of 2-cyanoiminothiazolidine
Procédé pour la préparation de la 2-cyanoiminothiazolidine

(30) Priorität: 04.08.1994 DE 4427539
(43) Veröffentlichungstag der Anmeldung: 03.04.1996
(73) Patentinhaber: SKW Trostberg Aktiengesellschaft, 83308 Trostberg (DE)
(72) Erfinder: Thalhammer, Franz, Dr., D-83308 Trostberg (DE)
(74) Vertreter: Huber, Bernhard, Dipl.-Chem.

(56) Entgegenhaltungen:
- DE-A- 2 205 745
- ORG. PREP. PROCED. IND., Bd. 23, Nr. 6, 1991, Seiten 721-728, XP002002935 ZMITEK, J. ET AL.: "The Synthesis and Reactions of N-Cayno-O-methylpseudoureas"
- J. HETEROCYCL. CHEM., Bd. 24, Nr. 1, 1987, Seiten 275-278, XP002002936 WEBB, R., W. ET AL.: "Diphenyl Cayncarbonimidate and Dichlorodiphenoxymethane as Synthones for the Construction of Heterocyclic Systems of Medical Interest"
- J. MED. CHEM., Bd. 38, Nr. 17, 1995, Seiten 3236-3245, XP002002937 ATWAL, K., S. ET AL.: "Cardioselective Anti-Ischemic ATP-Sensitive Potassium Chanel Openers. 3. Stucuture-Activity Studies on Benzopyranyl Cyanoguanidines: Modification of the Cyanoguanidine Portion"

## Beschreibung

Die Erfindung betrifft ein Verfahren zur Herstellung von 2-Cyaniminothiazolidin durch Reaktion von 2-Aminoethanthiol (Cysteamin) mit einem Dialkyl-N-cyanimidocarbonat.

Die Synthese von 2-Cyaniminothiazolidin unter Beteiligung von 2-Aminoethanthiol ist bekannt. So beschreibt die deutsche Offenlegungsschrift DE 22 05 745 neue, durch den N-Cyaniminorest substituierte Heterocyclen und Verfahren zu ihrer Herstellung, unter anderem die Umsetzung von 2-Aminoethanthiol mit Dialkyl-N-cyanimido-dithiocarbonaten. Dieses Verfahren weist aber den gravierenden Nachteil auf, daß während der Reaktion in zweifach stöchiometrischer Menge Mercaptane freigesetzt werden, die wegen ihrer Giftigkeit und ihres üblen Geruches nur äußerst schwierig und aufwendig gehandhabt werden können.

Die Verwendung von Diphenyl-N-cyanimidocarbonat zur Herstellung von 2-Cyaniminothiazolidin beschreiben R.L. Webb et al., in J. Heterocycl. Chem. 24, 275, 1987. Da das Diphenyl-N-cyanimidocarbonat als Ausgangsverbindung aber nicht in größerer Menge zur Verfügung steht und außerdem das bei der Reaktion frei werdende Phenol nur mit großem Aufwand vom Reaktionsprodukt vollständig abgetrennt werden kann, ist dieses Verfahren für eine technische Produktion des 2-Cyaniminothiazolidin ungeeignet.

Die Herstellung von 2-Cyaniminothiazolidin durch Reaktion von 2-Aminoethanthiol mit Dimethyl-N-cyanimidocarbonat in wäßriger Lösung wurde von I. Zmitek et al., in Org. Prep. Proced. Ind. 23, 721, (1991) beschrieben. Die lange Reaktionszeit und die dabei erzielte nur geringe Ausbeute von 48 % machen dieses Verfahren aber unwirtschaftlich. Außerdem geben die dort genannten physikalischen Daten Anlaß zum Zweifel, ob es sich bei dem isolierten und beschriebenen Produkt tatsächlich um 2-Cyaniminothiazolidin handelt.

Der Erfindung liegt daher die Aufgabe zugrunde, ein Verfahren zur Herstellung von 2-Cyaniminothiazolidin bereitzustellen, das in großtechnischem Maßstab durchführbar ist, dabei umweltrelevanten Aspekten Rechnung trägt und zu wirtschaftlich vertretbaren Ausbeuten führt, welches die genannten Mängel des Standes der Technik vermeidet.

Gelöst wird diese Aufgabe erfindungsgemäß durch ein Verfahren zur Herstellung von 2-Cyaniminothiazolidin der Formel 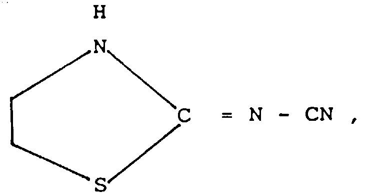 durch Reaktion von 2-Aminoethanthiol und einem Dialkyl-N-cyanimidocarbonat der allgemeinen Formel 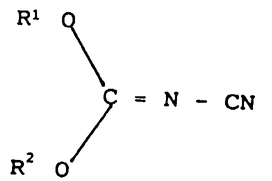 in der die Reste R¹ und R² gleich oder verschieden sein können und aliphatische Reste mit 1 bis 4 Kohlenstoffatomen darstellen, die auch zusammen eine Alkylenbrücke ausbilden können, welches dadurch gekennzeichnet ist, daß a) man 2-Aminoethanthiol und Dialkyl-N-cyanimidocarbonat bei Temperaturen zwischen -10 und +40°C im pH-Bereich zwischen 7 und 12 in Wasser und/oder einem organischen Lösemittel 30 Minuten bis 6 Stunden reagieren läßt und b) die Reaktion bei einem pH-Wert > 8 durch eine Temperaturerhöhung um 30 bis 60°C vervollständigt.

Es hat sich gezeigt, daß man das Produkt 2-Cyaniminothiazolidin nach diesem zweistufigen Verfahren in überraschend guten Ausbeuten und mit unerwartet hoher Reinheit herstellen kann.

Beim erfindungsgemäßen Verfahren wird zunächst 2-Aminoethanthiol (Cysteamin) in Wasser oder einem Gemisch aus Wasser und einem organischen Lösemittel gelöst, wobei als organische Lösemittel bevorzugt Alkohole mit 1 bis 4 Kohlenstoffatomen, wie Methanol und Ethanol oder auch Aceton eingesetzt werden. In einer alternativen Variante kann erfindungsgemäß auch ein Salz des 2-Aminoethanthiols vorgelegt werden; das freie Cysteamin wird dann durch Zugabe einer äquimolaren oder leicht überschüssigen Menge einer Base, beispielsweise Natronlauge, aus der Salzform freigesetzt, wofür das 2-Aminoethanthiol-hydrochlorid bevorzugt wird. Die Konzentration der Lösung des 2-Aminoethanthiols ist im allgemeinen kein kritischer Verfahrensparameter und somit in relativ weiten Grenzen variierbar. Vorzugsweise wird eine 1 bis 4 molare Lösung vorgelegt. Das Molverhältnis von 2-Aminoethanthiol zu Dialkyl-N-cyanimidocarbonat ist zweckmäßig 0,8 bis 1,2:1, bevorzugt etwa 1:1.

Dieser Lösung wird dann das Dialkyl-N-cyanimidocarbonat portionsweise oder kontinuierlich in fester oder gelöster Form zudosiert, wobei bevorzugt Dimethyl-N-cyanimidocarbonat eingesetzt wird.

Die Zugabereihenfolge der Reaktionspartner ist nicht limitierend und kann frei variiert werden, d.h. gemäß einer anderen Ausführungsform des erfindungsgemäßen Verfahrens kann 2-Aminoethanthiol auch zu einer vorgelegten Lösung des Dialkyl-N-cyanimidocarbonats zugegeben werden.

Als erfindungswesentlich ist unter anderem anzusehen, daß die eigentliche Reaktion in zwei aufeinanderfolgenden Schritten abläuft, wobei zunächst im Reaktionsschritt a) die Ausgangsverbindungen 2-Aminoethanthiol und Dialkyl-N-cyanimidocarbonat bei Temperaturen zwischen -10°C und +40°C zur Reaktion gebracht werden. Die Dosierrate für Dialkyl-N-cyanimidocarbonat wird dabei so gesteuert, daß die Temperatur des Reaktionsgemisches, gegebenenfalls unter äußerer Kühlung, den vorgegebenen Bereich nicht verläßt und vorzugsweise zwischen +10°C und +25°C liegt. Üblicherweise beträgt die Dauer der Dosierung bei dieser Vorgehensweise etwa 15 bis 30 Minuten. Es hat sich für das Verfahren im allgemeinen als sehr günstig erwiesen, wenn der Reaktionsschritt a) und auch der Reaktionsschritt b) unter einer Inertgasatmosphäre durchgeführt werden.

Im Anschluß an das Mischen der Ausgangsverbindungen wird während einer bevorzugten Reaktionszeit von 2 bis 4 Stunden bei der eingestellten Temperatur gerührt, wobei sich eine farblose Suspension des Primäradduktes bildet. Der pH-Wert der Lösung, der erfindungsgemäß während des Reaktionsschrittes a) im Bereich zwischen 7 und 12 liegt, wird nach dieser Zeit im Reaktionsschritt b) durch Zugabe einer Base auf > 8 eingestellt. Hierfür werden Alkalihydroxide, Alkalicarbonate und stark basische Amine wie Triethylamin und Dimethylamin bevorzugt, wobei diese Basen in einer besonders bevorzugten Variante in wäßriger Lösung zugegeben werden. Das Molverhältnis von zugesetzter Base und 2-Aminoethanthiol beträgt zweckmäßig 0,05 bis 1:1, bevorzugt 0,1 bis 0,2:1.

Nach der erfindungswesentlichen Temperaturerhöhung um 30 bis 60°C im Reaktionsschritt b) wird die Reaktionsmischung bevorzugt solange gerührt, bis das Primäraddukt analytisch nicht mehr nachweisbar und die Bildung des Thiazolidin-Rings vollständig ist. Die Dauer des Rührvorgangs ist dabei von der Reaktionstemperatur abhängig, die vorzugsweise 40 bis 50°C höher liegt als im Reaktionsschritt a); so werden üblicherweise bei 50°C 3 bis 4 Stunden und bei 80°C 1,5 bis 2 Stunden bis zum vollständigen Abreagieren des Primäradduktes benötigt.

Im Anschluß daran wird der pH-Wert des Reaktionsgemisches durch die Zugabe von Säure, bevorzugt von Salzsäure in den neutralen bis sauren Bereich gebracht, wobei sich ein leicht saurer pH-Wert von etwa pH 6 als optimal erwiesen hat, da bei diesen Bedingungen die Löslichkeit des Produktes ein Minimum durchläuft und so eine farblose Suspension erhalten wird.

Zur Vervollständigung der Produktabtrennung durch Kristallisation kann nötigenfalls auf eine Temperatur von < 20°C abgekühlt werden, wobei sich, falls eine sehr verdünnte Lösung vorliegt, ein zusätzlicher Einengschritt empfiehlt.

Die so erhaltene Suspension wird nach bekannten Verfahren, wie etwa Filtration, aufgetrennt und das erhaltene Produkt gegebenenfalls mit Wasser oder einem Wasser/Alkohol-Gemisch gewaschen. Abschließend kann das Feuchtprodukt getrocknet werden, wobei sich eine Trocknung bei Temperaturen von 50 bis 90°C als geeignet erwiesen hat.

Mit Hilfe des erfindungsgemäßen Verfahrens wird 2-Cyaniminothiazolidin in Ausbeuten von > 65 %, bevorzugt > 70 % und am meisten bevorzugt > 85 % in Form eines reinweißen Kristallpulvers von hoher Reinheit gewonnen.

Das erfindungsgemäße Verfahren wird durch die nachfolgenden Beispiele weiter erläutert.

### Beispiel 1

11,6 g (0,1 mol) 2-Aminoethanthiol-hydrochlorid wurden in einem Rührkolben unter Stickstoffatmosphäre in 50 ml Wasser gelöst und zu dieser Lösung bei Temperaturen zwischen 10 und 20°C innerhalb von 10 Minuten 16,0 g (0,1 mol) einer 25 %-igen (w/w) Natronlauge gegeben. Nachdem diese Lösung bei 20°C noch 30 Minuten gerührt worden war, wurden anschließend portionsweise 11,6 g (0,1 mol) Dimethyl-N-cyanimidocarbonat zugegeben. Dabei wurde durch äußere Kühlung die Temperatur bei ca. 20°C gehalten und 2,5 Stunden nachgerührt. Die entstandene Suspension wurde dann mit 2,0 g (0,02 mol) Triethylamin versetzt und die Temperatur auf 50°C erhöht. Nach 3 Stunden Rühren bei dieser Temperatur wurde mit 20 %-iger (w/w) Salzsäure ein pH-Wert von 6 eingestellt und die erhaltene Suspension nachfolgend auf 10°C abgekühlt. Über eine Filternutsche wurde der farblose Feststoff abgetrennt und zweimal mit je 10 ml Wasser gewaschen. Nach der Trocknung bei 50°C im Vakuum wurden 9,9 g (78 % der Theorie) eines farblosen Kristallpulvers erhalten, dessen Schmelzpunkt bei 153-155°C lag.

Durch HPLC wurde ein Reinheitsgrad von 99,9 % ermittelt. Aus der Mutterlauge lassen sich nach Einengen weitere 1,0 g des Produktes isolieren. Damit ergibt sich eine Gesamtausbeute von 86 % der Theorie.

### Beispiel 2

11,6 g (0,1 mol) 2-Aminoethanthiol-hydrochlorid wurden unter Stickstoff in 50 ml Wasser gelöst und innerhalb von 10 min. 16,0 g (0,1 mol) einer 25 %-igen (w/w) Natronlauge zudosiert. Zu dieser Lösung wurde bei 20°C eine Lösung aus 14,2 g (0,1 mol) Diethyl-N-cyanimidocarbonat und 20 ml Ethanol zugetropft und die entstehende Suspension 3 Stunden lang gerührt. Nach Zugabe von 2,0 g (0,02 mol) Triethylamin wurde 4 Stunden auf 60°C erwärmt. Anschließend wurde mit 20 %-iger (w/w) Salzsäure ein pH-Wert von 6,5 eingestellt und auf 10°C abgekühlt. Der ausgefallene Feststoff wurde abgesaugt, zweimal mit 10 ml Wasser gewaschen und getrocknet.

Auf diese Weise wurden 8,7 g (69 % der Theorie) Produkt mit einem Schmelzpunkt von 151-153°C und einer Reinheit von 99,1 % erhalten.

### Beispiel 3

7,8 g (0,1 mol) 2-Aminoethanthiol in 50 ml Wasser wurden mit 11,6 g (0,1 mol) Dimethyl-N-cyanimidocarbonat versetzt und 2,5 Stunden lang bei 20°C gerührt. Dann wurden 2,8 g (0,02 mol) Kaliumcarbonat zugesetzt und die Suspension 2 Stunden auf 80°C erwärmt.

Nach dem Abkühlen auf 10°C wurde mit Salzsäure ein pH-Wert von 5 eingestellt, der ausgefallene Feststoff abgesaugt und getrocknet.

Es wurden 8,9 g (70 % der Theorie) 2-Cyaniminothiazolidin mit einem Schmelzpunkt von 150-152°C und einer Reinheit von 99,0 % erhalten.

### Beispiel 4

Unter Stickstoffatmosphäre wurden 11,6 g (0,1 mol) 2-Aminoethanthiol-hydrochlorid in 25 ml Wasser mit 16,0 g (0,1 mol) einer 25 %-igen (w/w) Natronlauge bei einer Temperatur kleiner als 20°C versetzt, und die entstehende Lösung bei 20°C zu einer vorgelegten Suspension von 11,6 g (0,1 mol) Dimethyl-N-cyanimidocarbonat in Wasser getropft. Anschließend wurde noch 2 Stunden lang gerührt und die Suspension mit 1,5 g (0,02 mol) einer 60 %-igen (w/w) Lösung von Dimethylamin in Wasser versetzt. Nach der Zugabe des Amins wurde die Temperatur für 3 Stunden auf 50°C erhöht. Es wurde abgekühlt und mit wäßriger Salzsäure ein pH-Wert von 5 eingestellt. Die Suspension wurde noch 1 Stunde lang bei 10°C gerührt, der Feststoff durch Filtration abgetrennt und zweimal mit 10 ml Wasser gewaschen.

Nach Trocknung bei 50°C im Vakuum wurden 9,2 g (72 % der Theorie) 2-Cyaniminothiazolidin vom Schmelzpunkt 153-154°C und einer Reinheit nach HPLC von 99,3 % erhalten.

## Patentansprüche

1. Verfahren zur Herstellung von 2-Cyaniminothiazolidin der Formel 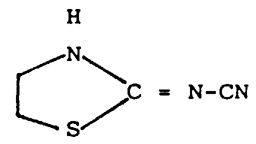 durch Reaktion von 2-Aminoethanthiol und einem Dialkyl-N-cyanimidocarbonat der allgemeinen Formel 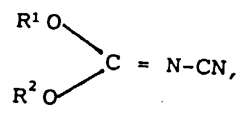 in der die Reste R¹ und R² gleich oder verschieden sein können und aliphatische Reste mit 1 bis 4 Kohlenstoffatomen darstellen, die auch zusammen eine Alkylenbrücke ausbilden können,
**dadurch gekennzeichnet**,
daß
a) man 2-Aminoethanthiol und Dialkyl-N-cyanimidocarbonat bei Temperaturen zwischen -10 und +40°C im pH-Bereich zwischen 7 und 12 in Wasser und/oder einem organischen Lösemittel 30 Minuten bis 6 Stunden reagieren läßt und
b) die Reaktion bei einem pH-Wert > 8 durch eine Temperaturerhöhung um 30 bis 60°C vervollständigt.

2. Verfahren nach Anspruch 1,
**dadurch gekennzeichnet**,
daß R¹ und R² je einen Methylrest darstellen.

3. Verfahren nach Anspruch 1 oder 2,
**dadurch gekennzeichnet,**
daß man als organisches Lösemittel Alkohole mit 1 bis 4 Kohlenstoffatomen oder Aceton einsetzt.

4. Verfahren nach einem der Ansprüche 1 bis 3,
**dadurch gekennzeichnet,**
daß das Molverhältnis von 2-Aminoethanthiol zu Dialkyl-N-cyanimidocarbonat 0,8 bis 1,2:1, bevorzugt 1:1, beträgt.

5. Verfahren nach einem der Ansprüche 1 bis 4,
**dadurch gekennzeichnet,**
daß das 2-Aminoethanthiol vor der Reaktion mit dem Dialkyl-N-cyanimidocarbonat durch Zugabe einer Base aus einem seiner Salze freigesetzt wird.

6. Verfahren nach Anspruch 5,
**dadurch gekennzeichnet,**
daß das Salz das Hydrochlorid ist.

7. Verfahren nach Anspruch 6,
**dadurch gekennzeichnet,**
daß die Base äquimolar oder in geringem Überschuß zugegeben wird.

8. Verfahren nach einem der Ansprüche 6 oder 7,
**dadurch gekennzeichnet,**
daß als Base Natronlauge zugegeben wird.

9. Verfahren nach einem der vorhergehenden Ansprüche,
**dadurch gekennzeichnet,**
daß die Temperatur im Reaktionsschritt a) zwischen +10 und +25°C liegt.

10. Verfahren nach einem der vorhergehenden Ansprüche,
**dadurch gekennzeichnet,**
daß die Reaktionszeit im Reaktionsschritt a) 2 bis 4 Stunden beträgt.

11. Verfahren nach einem der vorhergehenden Ansprüche,
**dadurch gekennzeichnet,**
daß der pH-Wert im Reaktionsschritt b) durch Basenzugabe auf > 8 eingestellt wird.

12. Verfahren nach einem der vorhergehenden Ansprüche,
**dadurch gekennzeichnet,**
daß man als Base im Reaktionsschritt b) Alkalihydroxide, Alkalicarbonate oder stark basische Amine in wäßriger Lösung verwendet.

13. Verfahren nach Anspruch 12,
**dadurch gekennzeichnet,**
daß die Base Triethylamin oder/und Diethylamin ist.

14. Verfahren nach Anspruch 12 oder 13,
**dadurch gekennzeichnet,**
daß das Molverhältnis von zugesetzter Base zum eingesetzten 2-Aminoethanthiol 0,05 bis 1:1 beträgt.

15. Verfahren nach Anspruch 14,
**dadurch gekennzeichnet,**
daß das Molverhältnis 0,1 bis 0,2:1 beträgt.

16. Verfahren nach einem der vorhergehenden Ansprüche,
**dadurch gekennzeichnet,**
daß die Temperatur im Reaktionsschritt b) um 40 bis 50°C erhöht wird.

17. Verfahren nach einem der vorhergehenden Ansprüche,
**dadurch gekennzeichnet,**
daß das Produkt im neutralen bis sauren pH-Bereich ausgefällt wird.

18. Verfahren nach Anspruch 17,
**dadurch gekennzeichnet,**
daß das Produkt bei pH 6 ausgefällt wird.

19. Verfahren nach einem der vorhergehenden Ansprüche,
**dadurch gekennzeichnet,**
daß man das Produkt durch Kristallisation bei einer Temperatur < 20°C abtrennt.

20. Verfahren nach einem der vorhergehenden Ansprüche,
**dadurch gekennzeichnet,**
daß man die Reaktionsschritte a) und b) unter Inertgas-Atmosphäre durchführt.

## Claims

1. Process for the production of 2-cyanoiminothiazolidine of formula 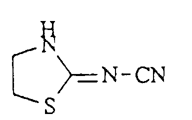 by reaction of 2-aminoethanethiol and a dialkyl-N-cyanoimidocarbonate of the general formula 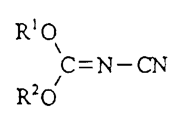 in which the residues R¹ and R² can be the same or different and represent aliphatic residues with 1 to 4 carbon atoms which together can also form an alkylene bridge,
**wherein**
a) 2-aminoethanethiol and dialkyl-N-cyanoimidocarbonate are allowed to react for 30 minutes to 6 hours at temperatures between -10 and +40°C in a pH range between 7 and 12 in water and/or an organic solvent and
b) the reaction is completed at a pH value of > 8 by increasing the temperature by 30 to 60°C.

2. Process as claimed in claim 1,
**wherein**
R¹ and R² each represent a methyl residue.

3. Process as claimed in claim 1 or 2,
**wherein**
alcohols with 1 to 4 carbon atoms or acetone are used as the organic solvent.

4. Process as claimed in one of the claims 1 to 3,
**wherein**
the molar ratio of 2-aminoethanethiol to dialkyl-N-cyanoimidocarbonate is 0.8 to 1.2:1, preferably 1:1.

5. Process as claimed in one of the claims 1 to 4,
**wherein**
the 2-aminoethanolthiol is released from one of its salts by addition of a base before the reaction with the dialkyl-N-cyanoimidocarbonate.

6. Process as claimed in claim 5,
**wherein**
the salt is a hydrochloride.

7. Process as claimed in claim 6,
**wherein**
the base is added in an equimolar amount or in a slight excess.

8. Process as claimed in one of the claims 6 or 7,
**wherein**
sodium hydroxide solution is added as the base.

9. Process as claimed in one of the previous claims,
**wherein**
the temperature in reaction step a) is between +10 and +25°C.

10. Process as claimed in one of the previous claims,
**wherein**
the reaction period in reaction step a) is 2 to 4 hours.

11. Process as claimed in one of the previous claims,
**wherein**
the pH value in reaction step b) is adjusted to > 8 by addition of a base.

12. Process as claimed in one of the previous claims,
**wherein**
alkali hydroxides, alkali carbonates or strongly basic amines in aqueous solution are used as the base in reaction step b).

13. Process as claimed in claim 12,
**wherein**
the base is triethylamine or/and diethylamine.

14. Process as claimed in claim 12 or 13,
**wherein**
the molar ratio of added base to 2-aminoethanethiol used is 0.05 to 1:1.

15. Process as claimed in claim 14,
**wherein**
the molar ratio is 0.1 to 0.2:1.

16. Process as claimed in one of the previous claims,
**wherein**
the temperature in reaction step b) is increased by 40 to 50°C.

17. Process as claimed in one of the previous claims,
**wherein**
the product is precipitated in a neutral to acidic pH range.

18. Process as claimed in claim 17,
**wherein**
the product is precipitated at pH 6.

19. Process as claimed in one of the previous claims,
**wherein**
the product is separated by crystallization at a temperature of < 20°C.

20. Process as claimed in one of the previous claims,
**wherein**
reaction steps a) and b) are carried out under an inert gas atmosphere.

## Revendications

1. Procédé de préparation de la 2-cyano-iminothiazolidine de formule 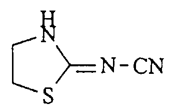 par réaction du 2-aminoéthanethiol et d'un N-cyano-imidocarbonate de dialkyle de formule générale 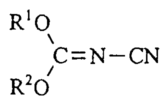 dans laquelle les restes R¹ et R² peuvent être identiques ou différents et représentent des restes aliphatiques de 1 à 4 atomes de carbone, qui peuvent aussi former ensemble un pont alkylène,
caractérisé en ce que
a) on fait réagir pendant 30 minutes à 6 heures le 2-aminoéthanethiol et le N-cyano-imidocarbonate de dialkyle à des températures comprises entre -10 et +40°C à un pH compris entre 7 et 12 dans de l'eau et/ou un solvant organique et
b) on complète la réaction à un pH supérieur à 8 en faisant monter la température de 30 à 60°C.

2. Procédé selon la revendication 1, caractérisé en ce que R¹ et R² représentent chacun un reste méthyle.

3. Procédé selon la revendication 1 ou 2, caractérisé en ce que l'on utilise comme solvant organique un alcool de 1 à 4 atomes de carbone ou l'acétone.

4. Procédé selon l'une des revendications 1 à 3, caractérisé en ce que le rapport molaire du 2-aminoéthanethiol au N-cyano-imidocarbonate de dialkyle est de 0,8 à 1,2:1, de préférence de 1:1.

5. Procédé selon l'une des revendications 1 à 4, caractérisé en ce que, avant la réaction avec le N-cyano-imidocarbonate de dialkyle, on libère le 2-aminoéthanethiol à partir d'un de ses sels en ajoutant une base.

6. Procédé selon la revendication 5, caractérisé en ce que le sel est le chlorhydrate.

7. Procédé selon la revendication 6, caractérisé en ce que l'on ajoute la base en une quantité équimolaire ou en un léger excès.

8. Procédé selon la revendication 6 ou 7, caractérisé en ce que l'on ajoute comme base une solution de soude.

9. Procédé selon l'une des revendications précédentes, caractérisé en ce que, dans l'étape de réaction a), la température est comprise entre +10 et +25°C.

10. Procédé selon l'une des revendications précédentes, caractérisé en ce que, dans l'étape de réaction a), le temps de réactionest de 2 à 4 heures.

11. Procédé selon l'une des revendications précédentes, caractérisé en ce que, dans l'étape de réaction b), on règle le pH à une valeur supérieure à 8 en ajoutant une base.

12. Procédé selon l'une des revendications précédentes, caractérisé en ce que, dans l'étape de réaction b), on utilise comme base un hydroxyde de métal alcalin, un carbonate de métal alcalin ou une amine fortement basique en solution aqueuse.

13. Procédé selon la revendication 12, caractérisé en ce que la base est la triéthylamine et/ou la diéthylamine.

14. Procédé selon la revendication 12 ou 13, caractérisé en ce que le rapport molaire de la base ajoutée au 2-aminoéthanethiol introduit est de 0,05 à 1:1.

15. Procédé selon la revendication 14, caractérisé en ce que le rapport molaire est de 0,1 à 0,2:1.

16. Procédé selon l'une des revendications précédentes, caractérisé en ce que, dans l'étape b), on fait monter la température de 40 à 50°C.

17. Procédé selon l'une des revendications précédentes, caractérisé en ce que l'on précipite le produit à un pH neutre à acide.

18. Procédé selon la revendication 17, caractérisé en ce que l'on précipite le produit à pH 6.

19. Procédé selon l'une des revendications précédentes, caractérisé en ce que l'on sépare le produit par cristallisation à une température inférieure à 20°C.

20. Procédé selon l'une des revendications précédentes, caractérisé en ce que l'on effectue les étapes de réaction a) et b) sous atmosphère de gaz inerte.
